# FASCICULE DE BREVET EUROPEEN

(11) **EP 1 152 022 B1**
(45) Date de publication et mention de la délivrance du brevet: **28.02.2007**
(21) Numéro de dépôt: 01401044.1
(22) Date de dépôt: 24.04.2001
(51) Int. Cl.: C08J 3/03, C08J 3/07, A61K 47/32, A61Q 19/00, A61K 9/107

(54) **Latex inverses sur squalane ou polyisobutène hydrogéné et compositions cosmétiques ou pharmaceutiques en comportant**
Inverslatex basierend auf Squalan oder hydriertem Polyisobuten und diese enthaltende kosmetische Zusammensetzungen, Hautkosmetika, sowie pharmazeutische Zusammensetzungen
Inverse latex based on squalane or hydrogenated polyisobutene and cosmetic compositions, dermocosmetics, dermopharmaceuticals or pharmaceutical compositions containing them

(30) Priorité: 05.05.2000 FR 0005790
(43) Date de publication de la demande: 07.11.2001
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Tabacchi, Guy, 81100 Castres (FR); Boiteux, Jean-Pierre, 81710 Saix (FR); Almaric, Chantal, 81700 Blan (FR); Michel, Nelly, 94700 Maisons Alfort (FR); Mallo, Paul, 75400 Chatou (FR)
(74) Mandataire: Conan, Philippe Claude

(56) Documents cités:
- EP-A- 1 010 708
- WO-A-00/32639
- FR-A- 2 721 511
- FR-A- 2 774 688
- FR-A- 2 774 996
- FR-A- 2 782 086
- FR-A- 2 785 801
- US-A- 5 185 395

## Description

La présente demande concerne des latex inverses eau dans huile, leur procédé de préparation et leur application en tant qu'épaississants et/ou émulsionnants pour des produits de soins de la peau et des cheveux et pour la fabrication de préparations cosmétiques, dermocosmétiques, dermopharmaceutiques ou pharmaceutiques.

Des polymères épaississants synthétiques, se présentant sous forme de latex inverses, sont décrits comme pouvant être utilisés dans la fabrication de compositions topiques, dans les demandes de brevet français publiées sous les numéros 2721511, 2733805, 2774688, 2774996 et 2782086 ainsi que dans la demande de brevet européen publiée sous le numéro EP 0 503 853. Parmi ceux-ci, la demande de brevet français 2 774 996 divulgue un latex inverse de poly[2-méthyl-2[(1-oxo-2-propényl) amino] 1-propanesulfonate de sodium] réticulé au méthylène bis(acrylamide) dans l'isohexadécane ; la demande de brevet français .2 782 086 divulgue un latex inverse de coplymère de 2-méthyl-2[(1-oxo-2-propényl) amino] 1-propanesulfonate de sodium et d'acrylate de 2-hydroxy éthyle] réticulé au méthylène bis(acrylamide) dans l'isohexadécane et la demande de brevet français 2 774 688 divulgue un latex inverse de copolymère de 2-méthyl-2[(1-oxo-2-propényl) amino] 1-propanesulfonate de sodium et d'acrylate de sodium réticulé au méthylène bis(acrylamide) dans l'isohexadécane.

Cependant, certains d'entre eux engendrent parfois des réactions d'intolérance de certaines peaux sensibles.

C'est pourquoi la demanderesse s'est intéressée à la recherche de nouvelles émulsions de polymères, qui soient mieux tolérées par la peau que celles de l'état de la technique.

L'invention a pour objet une composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché et/ou réticulé avec un composé diéthylénique ou polyéthylénique dans la proportion molaire exprimée par rapport aux monomères mis en oeuvre, de 0,005% à 1%, et de préférence de 0,01 % à 0,5 % et, plus particulièrement de 0,1 % à 0,25 %, caractérisé en ce que le solvant constitutif de la phase huile est choisi parmi le squalane ou le polyisobutène hydrogéné et le polyélectrolyte est,
ou bien un homopolymère à base d'un monomère possédant une fonction acide fort partiellement ou totalement salifiée ou bien un copolymère à base d'au moins un monomère possédant une fonction acide fort partiellement ou totalement salifiée, copolymérisé avec l'acrylamide et l'agent de réticulation et/ou de ramification est choisi parmi l'acide diallyloxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthylène bis(acrylamide) ou le triallylamine.

Le polyisobutène hydrogéné est commercialisé en France par la société Ets B. Rossow et Cie sous le nom PARLEAM - POLYSYNLANE™. Il est cité dans : Michel and Irene Ash ; Thesaurus of Chemical Products, Chemical Publishing Co , Inc. 1986 Volume I, page 211 (ISBN 0 7131 3603 0).

Le squalane est commercialisé en France par la société SOPHIM, sous le nom PHYTOSQUALAN™. Il est identifié dans Chemical Abstracts par le numéro RN = 111-01-3. C'est un mélange d'hydrocarbures contenant plus de 80% en poids de 2,6,10,15,19,23-hexaméthyl tétracosane.

Selon un deuxième aspect particulier de la présente invention, le solvant constitutif de la phase huile du latex inverse, est le polyisobutène hydrogéné.

Selon un troisième aspect particulier de la présente invention, le solvant constitutif de la phase huile du latex inverse, est le squalane.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition selon l'invention peut comporter des motifs réticulés et/ou des motifs branchés.

Par "agent émulsifiant du type eau dans huile", on désigne des agents émulsifiants possédant une valeur de HLB suffisamment faible pour fournir des émulsions eau dans huile tels que les polymères tensioactifs commercialisés sous le nom de HYPERMER™ ou tels que les esters de sorbitan, comme le monooléate de sorbitan commercialisé par la Société SEPPIC sous le nom de marque MONTANE 80™ ou l'isostéarate de sorbitan commercialisé par SEPPIC sous le nom de MONTANE 70™. On peut aussi adjoindre à ces agents émulsifiants, l'oléate de sorbitan éthoxylé avec 5 moles d'oxyde d'éthylène, commercialisé par la société SEPPIC sous le nom de MONTANOX™ 81.

Par "agent émulsifiant du type huile dans eau", on désigne des agents émulsifiants possédant une valeur de HLB suffisamment élevée pour fournir des émulsions huile dans l'eau tels que les esters de sorbitan éthoxylés comme l'oléate de sorbitan éthoxylé avec 20 moles d'oxyde d'éthylène, commercialisé par la société SEPPIC sous le nom de MONTANOX™80, l'huile de ricin éthoxylée à 40 moles d'oxyde d'éthylène commercialisée par la société SEPPIC sous le nom de SIMULSOL™ OL 50, le laurate de sorbitan éthoxylé à 20 moles d'oxyde d'éthylène commercialisé par la société SEPPIC sous le nom de MONTANOX™20 ou l'alcool laurique éthoxylé à 7 moles d'oxyde d'éthylène, commercialisé par la société SEPPIC sous le nom de SIMULSOL™ P7.

Comme agents émulsifiants possédant une valeur de HLB suffisamment élevée pour fournir des émulsions huile dans l'eau, il y a aussi les composés de formule (I)

R₁-O-[CH(R₂)-CH₂-O]ₙ-(G)ₓ-H (I),

dans laquelle R₁ représente un radical hydrocarboné linéaire ou ramifié, saturé ou insaturé comprenant de 1 à 30 atomes de carbones, R₂ représente un atome d'hydrogène ou radical alkyle comprenant 1 ou 2 atomes de carbone, G représente le reste d'un saccharide, x représente un nombre décimal compris entre 1 et 5, et n est égal, soit à zéro, soit à un nombre entier compris entre 1 et 30.

Par reste d'un saccharide, on désigne pour G, un radical bivalent résultant de l'enlèvement sur une molécule de sucre, d'une part d'un atome d'hydrogène d'un groupe hydroxyle et d'autre part du groupe hydroxyle anomérique. Le terme saccharide désigne notamment le glucose ou dextrose, le fructose, le mannose, le galactose, l'altrose, l'idose, l'arabinose, le xylose, le ribose, le gulose, le lyxose, le maltose, le maltotriose, le lactose, le cellobiose, le dextrane, le talose, l'allose, le raffinose, le lévoglucane, la cellulose ou l'amidon. La structure oligomérique (G)ₓ, peut se présenter sous toute forme d'isomérie, qu'il s'agisse d'isomérie optique, d'isomérie géométrique ou d'isomérie de position. Elle peut aussi représenter un mélange d'isomères.

Dans la formule (I) telle que définie précédemment, le radical :

R₁-O-[CH(R₂)-CH₂-O]ₙ-

est lié à G, par le carbone anomérique de manière à former une fonction acétal. Le groupe divalent -[CH(R₂)-CH₂-O]ₙ- représente, soit une chaîne composée uniquement de groupes éthoxyle (R₂ = H), soit une chaîne composée uniquement de groupes propoxyle (R₂ = CH₃), soit une chaîne composée à la fois de groupes éthoxyle et de groupes propoxyle. Dans ce dernier cas, les fragments -CH₂-CH₂-O- et -CH(CH₃)-CH₂-O- sont distribués dans ladite chaîne, de façon séquencée ou aléatoire.

Le nombre x, qui représente dans la formule (I) le degré moyen de polymérisation du saccharide, est plus particulièrement compris entre 1 et 3, notamment entre 1,05 et 2,5, tout particulièrement entre 1,1 et 2,0 et de préférence, inférieur ou égal à 1,5.

Comme agents tensioactifs émulsifiants possédant une valeur de HLB suffisamment élevée pour fournir des émulsions huile dans l'eau, il y a plus particulièrement, les composés dans formule (I) telle que définie précédemment dans laquelle G représente le reste du glucose ou le reste du xylose et/ou dans laquelle n est égal à 0, et/ou dans laquelle R₁ représente un radical comportant de 8 à 18 atomes de carbone et plus particulièrement dans laquelle R₁ représente un radical choisis parmi les radicaux octyle, décyle, undécyle, dodécyle, tétradécyle ou hexadécyle, lesdits radicaux étant linéaires ou ramifiés.

Comme produits commerciaux contenant lesdits composés, il y a par exemple :

Le SIMULSOL™SL8, commercialisé par la société SEPPIC, qui est une solution aqueuse contenant entre environ 35% et 45% en poids d'un mélange d'alkyl polyglycosides consistant en entre 45% en poids et 55% en poids d'un composé de formule (I), dans laquelle G représente le reste du glucose, x est égal à environ 1,45, n est égal à 0 et R₁ représente un radical décyle, et entre 45% en poids et 55% en poids d'un composé de formule (I), dans laquelle G représente le reste du glucose, x est égal à environ 1,45, n est égal à 0 et R₁ représente un radical octyle

Le SIMULSOL™ SL10, commercialisé par la société SEPPIC, qui est une solution aqueuse contenant entre environ 40% et poids et 50% en poids d'un mélange d'alkyl polyglycosides, consistant en environ 85% en poids d'un composé de formule (I), dans laquelle G représente le reste du glucose, x est égal à environ 1,45, n est égal à 0 et R₁ représente un radical décyle, environ 7,5% en poids d'un composé de formule (I), dans laquelle G représente le reste du glucose, x est égal à environ 1,45, n est égal à 0 et R₁ représente un radical dodécyle et environ 7,5% en poids d'un composé de formule (I), dans laquelle G représente le reste du glucose, le nombre x est égal à environ 1,45, n est égal à 0 et R₁ représente un radical tétradécyle ;

Le SIMULSOL™ SL11, commercialisé par la société SEPPIC, qui est une solution aqueuse contenant entre environ 40% et poids et 50% en poids d'un mélange d'alkyl polyglycosides de formule (I), dans laquelle G représente le reste du glucose, x est égal à environ 1,45, n est égal à 0 et R₁ représente un radical undécyle ; ou

Le SIMULSOL™ SL26, commercialisé par la société SEPPIC, qui est une solution aqueuse contenant entre environ 40% et poids et 55% en poids d'un mélange d'alkyl polyglycosides consistant en environ 70% en poids d'un composé de formule (I), dans laquelle G représente le reste du glucose, x est égal à environ 1,45, n est égal à 0 et R₁ représente un radical dodécyle, environ 25% en poids d'un composé de formule (I), dans laquelle G représente le reste du glucose, x est égal à environ 1,45, n est égal à 0 et R₁ représente un radical tétradécyle et environ 5% en poids d'un composé de formule (I), dans laquelle G représente le reste du glucose, x est égal à environ 1,45, n est égal à 0 et R₁ représente un radical hexadécyle.

La fonction acide fort du monomère en comportant est notamment la fonction acide sulfonique ou la fonction acide phosphonique, partiellement ou totalement salifiée. Ledit monomère peut être par exemple, l'acide styrènesulfonique partiellement ou totalement salifié. Il est de préférence l'acide 2-méthyl 2-[(1-oxo 2-propènyl) amino] 1-propanesulfonique partiellement ou totalement salifiée sous forme d'un sel de métal alcalin tel que par exemple le sel de sodium ou le sel de potassium, de sel d'ammonium ou d'un sel d'un amino alcool tel que par exemple le sel de monéthanolamine.

Selon un quatrième aspect particulier de la présente invention, le polyélectrolyte compris dans le latex inverse tel que défini précédemment est un copolymère comportant en proportions molaires de 30% à 50% d'acide 2-méthyl 2-[(1-oxo 2-propènyl) amino] 1-propanesulfonique (AMPS™) salifié sous forme de sel de sodium ou de sel d'ammonium et de 50% à 70% d'acrylamide.

Selon un cinquième aspect particulier de la présente invention, le polyélectrolyte compris dans le latex inverse tel que défini précédemment est un homopolymère d'acide 2-méthyl 2-[(1-oxo 2-propènyl) amino] 1-propanesulfonique salifié sous forme de sel de sodium ou de sel d'ammonium.

Le latex inverse tel que défini ci-dessus, contient généralement de 4% à 10% en poids, d'agents émulsifiants. Généralement de 20% à 50% et plus particulièrement de 25% à 40% du poids total des émulsifiants sont du type eau dans huile et de 80% à 50% et plus particulièrement de 75 à 60% sont du type huile dans eau.

Sa phase huile représente de 15% à 40%, et de préférence de 20% à 25%, de son poids total. Ce latex peut en outre contenir un ou plusieurs additifs choisis notamment parmi les agents complexants, les agents de transfert ou les agents limiteurs de chaînes.

L'invention a donc pour objet, une composition cosmétique, dermocosmétique, dermopharmaceutique ou pharmaceutique, caractérisée en ce qu'elle comprend au moins un composé épaississant, au moins un latex inverse tel que défini précédemment.

La composition cosmétique, dermocosmétique, dermopharmaceutique ou pharmaceutique définie ci-dessus comprend généralement de 0,1 % à 10 % et plus particulièrement entre 0,5% et 5% en poids dudit latex inverse. Elle se présente notamment, sous la forme d'un lait, d'une lotion, d'un gel, d'une crème, d'un savon, d'un bain moussant, d'un baume, d'un shampooing ou d'un après shampooing.

De façon générale, ledit latex inverse, peut remplacer avantageusement les produits vendus sous le nom SEPIGEL™ 305 ou SEPIGEL™ 501 par la demanderesse, dans les compositions cosmétiques, dermopharmaceutiques ou pharmaceutiques, car il présente aussi une bonne compatibilité avec les autres excipients utilisés pour la préparation de formulations telles que les laits, les lotions, les crèmes, les savons, les bains, les baumes, les shampooings ou les après shampooings. Il peut aussi être utilisé en combinaison lesdits SEPIGEL Il est notamment compatible avec les concentrés décrits et revendiqués dans les publications internationales WO 92/06778, WO 95/04592, W095/13863, WO 98/47610 ou FR 2734 496 ou avec les agents tensioactifs décrits dans WO 93/08204.

Il est particulièrement compatible avec le MONTANOV™ 68, le MONTANOV™ 82, le MONTANOV™ 202, le MONTANOV™WO18, le MONTANOV™ S ou le SEPIPERL™ N. Il peut également être utilisé dans des émulsions du type de celles décrites et revendiquées dans EP 0 629 396 et dans les dispersions aqueuses cosmétiquement ou physiologiquement acceptables avec un composé organo-polysiloxane choisi, par exemple parmi ceux décrits dans WO 93/05762 ou dans WO 93/21316. Il peut également être utilisé pour former des gels aqueux à pH acide cosmétiquement ou physiologiquement acceptables, tels que ceux décrit dans WO 93/07856 ; il peut encore être utilisé en association avec des celluloses non-ioniques, pour former par exemple des gels de coiffage, tels que ceux décrits dans EP 0 684 024 ou encore en association avec des esters d'acides gras et de sucre, pour former des compositions pour le traitement du cheveux ou de la peau telles que celles décrites dans EP 0 603 019. ou encore dans les shampooings ou après shampooings tels que décrits et revendiqués dans WO 92/21316 ou enfin en association avec un homo polymère anionique tels que le CARBOPOL™ pour former des produits de traitement des cheveux comme ceux décrits dans DE 195 23596. Il est également compatible avec de nombreux principes actifs, tels que par exemple, les agents autobronzants comme le dihydroxyacétone (DHA) ou les agents anti-acné ; il peut donc être introduit dans des compositions auto-bronzantes comme celles revendiquées dans EP 0 715 845, EP 0604249,
EP 0576188 ou dans WO 93/07902. Il est également compatible avec les dérivés N-acylés d'aminoacides, ce qui permet son utilisation dans des compositions apaisantes notamment pour peaux sensibles, telles que celles décrites ou revendiquées dans WO 92/21318, WO 94/27561 ou WO 98/09611. Il est aussi compatible avec les acides glycoliques, avec l'acide lactique, avec l'acide salicylique les rétinoïdes, le phénoxy éthanol, ies sucres, le glycéraldéhyde, les xanthanes, les acides de fruit, et les divers polyols utilisés dans la fabrication de formulations cosmétiques.

L'invention a donc aussi pour objet, l'utilisation d'un latex inverse tel que défini précédemment, pour préparer une composition cosmétique, dermocosmétique, dermopharmaceutique ou pharmaceutique.

Les exemples qui suivent ont pour but d'illustrer la présente invention sans toutefois la limiter. Ils montrent que les nouveaux latex inverses n'irritent pas la peau et que leurs propriétés physiques permettent leur utilisation dans la préparation de compositions cosmétiques, dermopharmaceutiques ou pharmaceutiques plus particulièrement destinées au traitement des peaux sensibles.

### A) Exemples de préparations de compositions selon l'invention

### Exemple 1: Latex inverse d'un copolymère AMPS/acrylamide réticulé au méthylène bis(acrylamide), dans le squalane (Composition 1).

**a) -** On charge dans un bécher sous agitation :
   - 288,0 g d'une solution commerciale d'acrylamide à 50%,
   - 363,0 g d'une solution commerciale à 55% de 2-méthyl 2-[(1-oxo 2-propènyl) amino] 1-propanesulfonate de sodium,
   - 0,45 g d'une solution aqueuse commerciale à 40% en poids de diéthylènetriaminepentacétate de sodium et
   - 0,09 g de méthylène bis(acrylamide).

   Le pH de cette solution aqueuse est ajustée à 5,0 par ajout d'environ 0,4 g d'acide 2-méthyl 2-[(1-oxo 2-propènyl) amino] 1-propanesulfonique.
**b**) - On prépare une phase organique en mélangeant :
   - 247,7 g de squalane,
   - 19,5 g de MONTANE™ 80 VG,
   - 11,5g de MONTANOX™ 81 VG et
   - 0,27 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 10 ml d'une solution contenant 0,28% en poids, d'hydroperoxyde de cumène dans le squalane, puis, après homogénéisation de la solution, une solution aqueuse de métabisulfite de sodium (2,5 g dans 100 ml d'eau) à raison de 0,5 ml/minute pendant environ 60 minutes et en laissant monter la température jusqu'à la température de polymérisation. On maintient alors le milieu réactionnel pendant environ 90 minutes à cette température, à l'issu desquelles le mélange obtenu est refroidi jusqu'à 35° environ. On introduit lentement 48,1 g d'oléate de sorbitan éthoxylé à 20 moles (MONTANOX™ 80) et on obtient l'émulsion eau dans huile désirée.

### Propriétés physiques

Viscosité dans l'eau à 3% du latex (Brookfield RVT Mobile 6, vitesse 5) :
η = 100 000 mPas

Viscosité dans l'eau à 3% du latex (Brookfield RVT Mobile 6, vitesse 20) :
η = 33 800 mPas.

### Exemple 2 : Latex inverse d'un copolymère AMPS/acrylamide réticulé au méthylène bis(acrylamide), dans le polyisobutène hydrogéné (composition 2)

**a) -** On charge dans un bécher sous agitation :
   - 288,0 g d'une solution commerciale d'acrylamide à 50%,
   - 363,0 g d'une solution commerciale à 55% de 2-méthyl 2-[(1-oxo 2-propènyl) amino] 1-propanesulfonate de sodium,
   - 0,45 g d'une solution aqueuse commerciale à 40% en poids de diéthylènetriaminepentacétate de sodium et
   - 0,09 g de méthylène bis(acrylamide).

   Le pH de cette solution aqueuse est ajustée à 5,0 par ajout d'environ 0,4 g d'acide 2-méthyl 2-[(1-oxo 2-propènyl) amino] 1-propanesulfonique.
**b**) - On prépare une phase organique en mélangeant:
   - 235,5 g de polyisobutène hydrogéné,
   - 15,1 g de MONTANE™ 80 VG,
   - 7,4 g de MONTANOX™ 81 VG et
   - 0,25 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 10 ml d'une solution contenant 0,28% en poids, d'hydroperoxyde de cumène dans le polyisobutène hydrogéné, puis, après homogénéisation de la solution, une solution aqueuse de métabisulfite de sodium (2,5g dans 100 ml d'eau) à raison de 0,5 ml /minute pendant environ 60 minutes et en laissant monter la température jusqu'à la température de polymérisation. On maintient alors le milieu réactionnel pendant environ 90 minutes à cette température, à l'issu desquelles le mélange obtenu est refroidi jusqu'à 35° environ. On introduit lentement 25 g quelles le mélange obtenu est refroidi jusqu'à 35° environ. On introduit lentement 25 g d'oléate de sorbitan éthoxylé à 20moles (MONTANOX™ 80) et on obtient l'émulsion eau dans huile désirée.

### Propriétés physiques

Viscosité dans l'eau à 3% du latex (Brookfield RVT Mobile 6, vitesse 5) :
η = 120 000 mPas

Viscosité dans l'eau à 3% du latex (Brookfield RVT Mobile 6, vitesse 20) :
η = 37 000 mPas.

### Exemple 3 : Latex inverse d'un homopolymère AMPS réticulé au triallylamine, dans le squalane (Composition 3)

**a) -** On charge dans un bécher sous agitation:
   - 660 g d'une solution commerciale à 55% de 2-méthyl 2-[(1-oxo 2-propènyl) amino] 1-propanesulfonate de sodium,
   - 0,45 g d'une solution aqueuse commerciale à 40% en poids de diéthylènetriaminepentacétate de sodium et
   - 0,5 g de triallylamine.

   Le pH de cette solution aqueuse est ajustée à 5,0 par ajout d'environ 0,4 g d'acide 2-méthyl 2-[(1-oxo 2-propènyl) amino] 1-propanesulfonique.
**b**) - On prépare une phase organique en mélangeant :
   - 247,7 g de squalane
   - 19,5 g de MONTANE™ 80 VG,
   - 11,5 g de MONTANOX™ 81 VG et
   - 0,27 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 10 ml d'une solution contenant 0,28 % en poids, d'hydroperoxyde de cumène dans le squalane, puis, après homogénéisation de la solution, une solution aqueuse de métabisulfite de sodium (2,5 g dans 100 ml d'eau) à raison de 0,5 ml /minute pendant environ 60 minutes et en laissant monter la température jusqu'à la température de polymérisation. On maintient alors le milieu réactionnel pendant environ 90 minutes à cette température, à l'issu desquelles le mélange obtenu est refroidi jusqu'à 35° environ. On introduit lentement 48,1 g d'oléate de sorbitan éthoxylé à 20moles (MONTANOX™ 80) et on obtient l'émulsion eau dans huile désirée.

### Propriétés physiques

Viscosité dans l'eau à 3% du latex (Brookfield RVT Mobile 6, vitesse 5) :
η = 70 000 mPas

Viscosité dans l'eau à 3% du latex (Brookfield RVT Mobile 6, vitesse 20) :
η = 20 000 mPas.

### Exemple 4 : Latex inverse d'un copolymère AMPS/acrylamide réticulé au triallylamine dans le squalane (Composition 4).

On opère comme à l'exemple 1, mais en remplaçant les 0,09 g de méthylène bis(acrylamide) par 1,01 g de triallylamine et les 48,1 g de MONTANOX™ 80 par 40 g de SIMULSOL™ SL8 et l'on obtient l'émulsion eau dans huile désirée.

### Propriétés physiques

Viscosité dans l'eau à 3% du latex (Brookfield RVT Mobile 6, vitesse 5) :
η = 105 000 mPas

Viscosité dans l'eau à 3% du latex (Brookfield RVT Mobile 6, vitesse 20) :
η = 34 000 mPas.

### B Propriétés des compositions selon l'invention

### a) Etude comparative de la tolérance cutanée

La tolérance locale épicutanée d'une série de gels-crèmes, contenant 3 % et 5 % en poids d'une des compositions 1 à 4 préparées comme décrit ci-dessus, a été déterminée et comparée à celle observée avec un latex inverse d'un copolymère AMPS/acrylamide réticulé au méthylène bis(acrylamide), dans l'ISOPAR™ M (Composition A). selon le protocole suivant :

On applique la composition à tester, une surface d'environ 50 mm² de la région sous-capulaire gauche, de la peau (de type "peau japonaise") du dos de 19 volontaires sains. Le contact est maintenu pendant 48 heures sous timbre occlusif.

Cette application est aussi réalisée dans les mêmes conditions avec un timbre seul (sans composition) en tant que témoin négatif.

L'observation clinique de la surface de peau ainsi traitée est réalisée 30 minutes puis 24 heures après la dépose desdits timbres. Ces observations sont faites par comparaison à la surface non traitée témoin négatif.

La quantification de l'irritation cutanée, selon une échelle numérique allant de 0 à 4 (0 : pas d'effet ; 1 : effet très léger ; 2 : effet net ; 3 et 4: effet modéré à sévère selon les réactions), est réalisée pour chacune des réactions éventuellement observées à savoir : érythème, oedème, vésicules, sécheresse de !a peau, rugosité de la peau et la réflectivité de la peau.

Les indices de tolérance cutanée (IC), consignés dans le tableau suivant, expriment la moyenne de la somme des effets quantifiés relevés sur chaque volontaire :
IC = 0 signifie qu'aucune irritation n'a été observée,
IC = 0,5 signifie que le produit est statistiquement bien toléré,
IC > 0,5 signifie que le produit engendre une intolérance.

| | indice de tolérance cutanée | |
|---|---|---|
| | gel à 3% | gel à 5% |
| Composition 1 | 0,11 | 0,21 |
| Composition 2 | 0,89 | 0,63 |
| Composition A | 2,42 | 3,20 |

Ces résultats montrent que de façon inattendue, le squalane, le polyisobutène hydrogéné, l'isohexadécane et le MARCOL™52, potentialisent la tolérance cutanée polymère du latex inverse.

### C) Exemples de formulations préparées avec les compositions selon l'invention

### Exemple 5 : gel solaire non gras

### FORMULE

| | | |
|---|---|---|
| A | Composition 3: | 3,00% |
| | Eau : | 30% |
| B | SEPICIDE™ Cl : | 0,20% |
| | SEPICIDE™ HB : | 0,30% |
| | Parfum : | 0,10% |
| C | colorant : | q.s.p. |
| | eau: | 30% |
| D | MICROPEARL™ M 100 : | 3,00% |
| | Eau : | q.s.p. 100% |
| E | huile de silicone : | 2,0% |
| | PARSOL™ MCX : | 5,00% |

### MODE OPERATOIRE

Introduire B dans A; ajouter C, puis D, puis E.

### Exemple 6 : Lait solaire

### FORMULE

| | | |
|---|---|---|
| A | MONTANOV™ S : | 3,0% |
| | huile de sésame : | 5,0% |
| | PARSOL™ MCX : | 5,0% |
| | Carraghénane λ : | 0,10% |
| B | eau : | q.s.p. 100% |
| C | Composition 1 : | 0,80% |
| D | Parfum: | q. s. |
| | Conservateur : | q. s. |

### MODE OPERATOIRE

Emulsionner B dans A à 75°C puis ajouter C vers 60°C, puis D vers 30°C et ajuster le pH si nécessaire

### Exemple 7 : Gel de massage

### FORMULE

| | | |
|---|---|---|
| A | Composition 2 : | 3,5% |
| | Eau : | 20,0% |
| B | colorant : | 2 gouttes/100g |
| | Eau : | q.s. |
| C | alcool: | 10% |
| | Menthol : | 0,10% |
| D | huile de silicone : | 5,0% |

### MODE OPERATOIRE

Ajouter B dans A, puis ajouter au mélange, C puis D

### Exemple 8 : Gel coup d'éclat

### FORMULE

| | | |
|---|---|---|
| A | Composition 4: | 4% |
| | Eau : | 30% |
| B | ELASTINE HPM : | 5,0% |
| C | MICROPEARL™ M 100 : | 3% |
| | Eau : | 5% |
| D | SEPICIDE™ Cl : | 0,2% |
| | SEPICIDE™ HB : | 0,3% |
| | Parfum : | 0,06% |
| Sodium pyrolidinonecarboxylate 50% : 1% | | |
| Eau : | | q.s.p. 100% |

### MODE OPERATOIRE

Préparer A ; additionner B, puis C, puis D.

### Exemple 9 : Lait corporel

### FORMULE

| | |
|---|---|
| MONTANOV™ S : | 3,5% |
| LANOL™ 37T : | 8,0% |
| SOLAGUM™ L : | 0,05% |
| Eau: | q.s.p.100% |
| Benzophénone: | 2,0% |
| diméthicone 350cPs : | 0,05% |
| Composition 3 : | 0,8% |
| conservateur : | 0,2% |
| parfum : | 0,4% |

### Exemple 10 : Crème aux AHA pour peaux sensibles

### FORMULE

| | |
|---|---|
| mélange de lauryl aminoacides : | 0,1% à 5% |
| aspartate de magnésium et de potassium: | 0,002% à 0,5% |
| LANOL™ 99 : | 2% |
| MONTANOV™ 68 : | 5,0% |
| Eau : | q.s.p. 100% |
| Composition 2 : | 1,50% |
| acide gluconique : | 1,50% |
| tri éthylamine : | 0,9% |
| SEPICIDE™ HB : | 0,3% |
| SEPICIDE™ CI : | 0,2% |
| Parfum : | 0,4% |

### Exemple 11 : Lait démaquillant

### FORMULE

| | |
|---|---|
| SEPIPERL™ N : | 3% |
| PRIMOL™ 352 : | 8,0% |
| huile d'amandes douces : | 2 % |
| eau : | q.s.p. 100% |
| Composition 2 : | 0,8% |
| conservateur : | 0,2% |

### Exemple 12 : Emulsion fluide à pH alcalin

| | |
|---|---|
| MARCOL™ 82 : | 5,0% |
| NaOH : | 10,0% |
| Eau : | q.s.p. 100% |
| Composition 1 : | 1,5% |

### Exemple 13 : Fond de teint fluide

### FORMULE

| | |
|---|---|
| SIMULSOL™ 165 : | 5,0% |
| LANOL™ 84D : | 8,0% |
| LANOL™ 99 : | 5,0% |
| Eau : | q.s.p. 100% |
| pigments et charges minérales : | 10,0% |
| Composition 1 : | 1,2% |
| conservateur : | 0,2% |
| parfum : | 0,4% |

### Exemple 14 : Gel contour des yeux

### FORMULE

| | |
|---|---|
| Composition 1 : | 2,0% |
| Parfum: | 0,06% |
| Sodium pyrrolidinonecarboxylate : | 0,2% |
| DOW CORNING™ 245 Fluid: | 2,0% |
| Eau: | q.s.p. 100% |

### Exemple 15 : Composition de soin non rincée

### FORMULE

| | |
|---|---|
| Composition 4 : | 1,5% |
| Parfum : | q.s |
| Conservateur : | q.s |
| DOW CORNING™ X2 8360 : | 5,0% |
| DOW CORNING™ Q2 1401 : | 15,0% |
| Eau : | q.s.p. 100% |

### Exemple 16 : Gel amincissant

| | |
|---|---|
| Composition 4 : | 5% |
| Ethanol : | 30% |
| Menthol : | 0,1% |
| Caféine: | 2,5% |
| extrait de ruscus : | 2% |
| extrait de lierre : | 2% |
| SEPICIDE™ HP : | 1% |
| Eau: | q.s.p. 100% |

### Exemple 17 : Gel crème teinté ultra naturel

### FORMULE

| | | |
|---|---|---|
| A | eau : | 10,0% |
| | Butylène glycol : | 4,0% |
| | PEG-400 : | 4,0% |
| | PECOSIL™ PS100 : | 1,5% |
| | NaOH : | q.s. pH = 7 |
| | Dioxyde de titane : | 2,0% |
| | Oxyde de fer jaune : | 0,8% |
| | Oxyde de fer rouge : | 0,3% |
| | Oxyde de fer noir : | 0,05% |
| B | LANOL™ 99 : | 4,0% |
| | Caprylic capric triglycéride | 4,0% |
| | SEPIFEEL™ ONE : | 1,0% |
| | Composition 2 : | 3,0% |
| C | eau : | q.s.p. 100% |
| | MICROPEARL™ M305 : | 2,0% |
| | EDTA tétrasodé : | 0,05% |
| | Cyclométhicone : | 4,0% |
| D | SEPICIDE™ HB : | 0,5% |
| | SEPICIDE CI : | 03% |
| | Parfum : | 0,2% |

### MODE OPERATOIRE

préparer le mélange B + C puis ajouter A puis D.

### Exemple 18 : Crème aux AHA

### FORMULE

| | | |
|---|---|---|
| A | MONTANOV™ 68 : | 5,0% |
| | LIPACIDE™ PVB: | 1,05% |
| | LANOL™ 99 : | 10,0% |
| B | eau: | q.s.p. 100% |
| | Acide gluconique : | 1,5% |
| | TEA (triéthanolamine) : | 0,9% |
| C | Composition 2 : | 1,5% |
| D | parfum: | 0,4% |
| | SEPICIDE™ HB: | 0,2% |
| | SEPICIDE™ CI: | 0,4% |

### Exemple 19 : Lait solaire au monoï de Tahiti

### FORMULE

| | | |
|---|---|---|
| A | Monoï de Tahiti : | 10% |
| | LIPACIDE™ PVB : | 0,5% |
| | Composition 3 : | 2,2% |
| B | eau : | q.s.p. 100% |
| C | parfum : | 0,1% |
| | SEPICIDE™ HB : | 0,3% |
| | SEPICIDE™ CI: | 0,1% |
| | PARSOL™ MCX : | 4,0% |

### Exemple 20: Soin solaire pour le visage

### FORMULE

| | | |
|---|---|---|
| A | Cyclométhicone et diméthiconol : | 4,0% |
| | Composition 2 : | 3,5% |
| B | eau : | q.s.p. 100% |
| C | parfum : | 0,1% |
| | SEPICIDE™ HB : | 0,3% |
| | SEPICIDE™ CI : | 0,21% |
| | PARSOL™ MCX : | 5,0% |
| | Micatitane : | 2,0% |
| | Acide lactique : | q.s.p. pH = 6,5 |

Les caractéristiques des produits utilisés dans les exemples précédents, sont les suivantes :
Le MONTANOV™ 68 (cétéaryl glucoside, alcool cétéarylique), est une composition auto-émulsionnable telle que celles décrites dans WO 92/06778, commercialisée par la société SEPPIC.
Le MONTANOV™ 202 (arachidyl glucoside, alcool arachydilique + alcool béhènylique), est une composition auto-émulsionnable telle que celles décrites dans WO 98/17610, commercialisée par la société SEPPIC.
Le MICROPEARL™ M 305 est une poudre hydrodispersible soyeuse à base de copolymer méthylméthacrylate réticulé.
Le MICROPEARL™ M 100 est une poudre ultra fine au toucher très doux et à action matifiante commercialisée par la société MATSUMO
Le SEPICIDE™ Cl, imidazoline urée, est un agent conservateur commercialisé par la société SEPPIC.
PEMULEN™ TR est un polymère acrylique commercialisé par GOODRICH.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL™ S sont des facteurs de consistance commercia MERCK.
Le DOW CORNING™ 245 Fluid est de la cyclométhicone, commercialisée par la société DOW CORNING.
Le LIPACIDE™ PVB,est un hydrolysat de protéines de blé palmitoylé, est commercialisée par la société SEPPIC.
Le SEPICONTROL™ A5 est un mélange capryloyl glycine, sarcosine, extrait de cinnamon zylanicum, commercialisé par la société SEPPIC, tel que ceux décrits dans la demande internationale de brevet PCT/FR98/01313 déposée le 23 juin 1998.
Le CAPIGEL™ 98 est un copolymère d'acrylates commercialisé par la société SEPPIC.
Le LANOL™ 2681 est un mélange caprylate, caprate de coprah, commercialisé par la société SEPPIC.

## Revendications

1. Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché et/ou réticulé avec un composé diéthylénique ou polyéthylénique dans la proportion molaire exprimée par rapport aux monomères mis en oeuvre, de 0,005% à 1 %, et de préférence de 0,01 % à 0,5 % et, plus particulièrement de 0,1 % à 0,25 %. **caractérisé en ce que** le solvant constitutif de la phase huile est choisi parmi le squalane ou le polyisobutène hydrogéné et le polyélectrolyte est,
ou bien un homopolymère à base d'un monomère possédant une fonction acide fort partiellement ou totalement salifiée ou bien un copolymère à base d'au moins un monomère possédant une fonction acide fort partiellement ou totalement salifiée, copolymérisé avec l'acrylamide et l'agent de réticulation et/ou de ramification est choisi parmi l'acide diallyloxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthylène bis(acrylamide) ou le triallylamine.

2. Composition telle que définie à la revendication 1, **caractérisé en ce que** le solvant constitutif de la phase huile est le polyisobutène hydrogéné.

3. Composition telle que définie à la revendication 1, **caractérisé en ce que** le solvant constitutif de la phase huile est le squalane.

4. Composition telle que définie à l'une quelconques des revendications 1 à 3, dans laquelle le ou les agents émulsifiants du type eau dans huile, sont choisis parmi le monooléate de sorbitan, l'isostéarate de sorbitan ou l'oléate de sorbitan éthoxylé avec 5 moles d'oxyde d'éthylène.

5. Composition telle que définie à l'une quelconques des revendications 1 à 4, dans laquelle le ou les agents émulsifiants du type huile dans eau sont choisis parmi l'oléate de sorbitan éthoxylé avec 20 moles d'oxyde d'éthylène, l'huile de ricin éthoxylée à 40 moles d'oxyde d'éthylène, le laurate de sorbitan éthoxylé à 20 moles d'oxyde d'éthylène, l'alcool laurique éthoxylé à 7 moles d'oxyde d'éthylène.

6. Composition telle que définie à l'une quelconques des revendications 1 à 5, dans laquelle le ou les agents émulsifiants du type huile dans eau sont choisis parmi les composés de formule (I)
R₁-O-[CH(R₂)-CH₂-O]ₙ-(G)ₓ-H (I)
dans laquelle R₁ représente un radical hydrocarboné linéaire ou ramifié, saturé ou insaturé comprenant de 1 à 30 atomes de carbones, R₂ représente un atome d'hydrogène ou radical alkyle comprenant 1 ou 2 atomes de carbone, G représente le reste d'un saccharide, x représente un nombre décimal compris entre 1 et 5, et n est égal, soit à zéro, soit à un nombre compris entre1 et 30.

7. Composition telle que définie à la revendication 6, pour laquelle, dans la formule (I), le nombre x est compris entre 1 et 3, plus particulièrement entre 1,05 et 2,5, tout particulièrement entre 1,1 et 2,0 et de préférence, inférieur ou égal à 1,5.

8. Composition telle que définie à l'une des revendications 6 ou 7 pour laquelle, dans la formule (I), G représente le reste du glucose ou le reste du xylose et n est égal à 0.

9. Composition telle que définie à l'une quelconque des revendications 6 à 8, pour laquelle, dans la formule (I), R₁ représente un radical comportant de 8 à 18 atomes de carbone et plus particulièrement un radical octyle, décyle, undécyle, dodécyle, tétradécyle ou hexadécyle, lesdits radicaux étant linéaires ou ramifiés.

10. Composition telle que définie à l'une quelconque des revendications 1 à 9 pour laquelle, la fonction acide fort du monomère en comportant est la fonction acide sulfonique ou la fonction acide phosphonique partiellement ou totalement salifiée.

11. Composition telle que définie à la revendication 10, pour laquelle le monomère à fonction acide fort est l'acide 2-méthyl 2-[(1-oxo 2-propènyl) amino] 1-propanesulfonique partiellement ou totalement salifiée sous forme de sel de sodium, de potassium, d'ammonium ou de monoéthanolamine.

12. Composition telle que définie à l'une quelconque des revendications 1 à 11, dans laquelle le polyélectrolyte est un copolymère comportant en propor-tions molaires de 30% à 50% d'acide 2-méthyl 2-[(1-oxo 2-propènyl) amino] 1-propanesulfonique salifié sous forme de sel de sodium ou de sel d'ammonium et de 50% à 70% d'acrylamide.

13. Composition telle que définie à l'une quelconque des revendications 1 à 11, dans laquelle le polyélectrolyte est un homopolymère d'acide 2-méthyl 2-[(1-oxo 2-propènyl) amino] 1-propanesulfonique salifié sous forme de sel de sodium ou de sel d'ammonium.

14. Composition telle que définie à l'une quelconque des revendications 1 à 13, **caractérisée en ce qu'**elle contient de 4% à 10% en poids, d'agents émulsifiants.

15. Composition telle que définie à la revendication 14, dans laquelle de 20% à 50% et plus particulièrement de 25% à 40% du poids total des émulsifiants sont du type eau dans huile et de 80% à 50% et plus particulièrement de 75 à 60% en poids sont du type huile dans eau.

16. Composition telle que définie à l'une quelconque des revendications 1 à 15, **caractérisée en ce que** la phase huile représente de 15% à 40%, et de préférence de 20% à 25%, de son poids total.

17. Composition telle que définie à l'une quelconque des revendications 1 à 16, **caractérisée en ce qu'**elle comporte en outre, un ou plusieurs additifs choisis parmi les agents complexant, les agents de transfert ou les agents limiteurs de chaînes.

18. Composition cosmétique, dermocosmétique, dermopharmaceutique ou pharmaceutique, **caractérisée en ce qu'**elle comprend de 0,1 % à 10 % en poids, de la composition telle que définie à l'une quelconque des revendications 1 à 16.

19. Composition cosmétique, dermocosmétique, dermopharmaceutique ou pharmaceutique telle que définie à la revendication 18, sous la forme d'un lait, d'une lotion, d'un gel, d'une crème, d'un savon, d'un bain moussant, d'un baume, d'un shampooing ou d'un après shampooing.

20. Utilisation d'une composition telle que définie à l'une des revendications 1 à 17, pour préparer des compositions cosmétiques, dermocosmétiques, dermopharmaceutiques ou pharmaceutiques.

## Claims

1. Composition comprising an oil phase, an aqueous phase, at least one emulsifying agent of water-in-oil (W/0) type, at least one emulsifying agent of oil-in-water (O/W) type, in the form of a self-invertible inverse latex comprising from 20% to 70% by weight and preferably from 25% to 50% by weight of a polyelectrolyte which is branched and/or crosslinked with a diethylenic or polyethylenic compound in the molar proportion, expressed with respect to the monomers employed, of 0.005% to 1%, preferably of 0.01% to 0.5% and more particularly of 0.1% to 0.25%, **characterized in that** the constituent solvent of the oil phase is chosen from squalane or hydrogenated polyisobutene and the polyelectrolyte is,
either a homopolymer based on a monomer having a partially or completely salified strong acid functional group or a copolymer based on at least one monomer having a partially or completely salified strong acid functional group copolymerized with acrylamide, and the crosslinking and/or branching agent is chosen from diallyloxyacetic acid or one of its salts, such as sodium diallyloxyacetate, ethylene glycol dimethacrylate, ethylene glycol diacrylate, diallylurea, trimethylolpropane triacrylate, methylenebis(acrylamide) or triallylamine.

2. Composition as defined in Claim 1, **characterized in that** the constituent solvent of the oil phase is hydrogenated polyisobutene.

3. Composition as defined in Claim 1, **characterized in that** the constituent solvent of the oil phase is squalane.

4. Composition as defined in any one of Claims 1 to 3, in which the emulsifying agent or agents of the water-in-oil type are chosen from sorbitan monooleate, sorbitan isostearate or sorbitan oleate ethoxylated with 5 mol of ethylene oxide.

5. Composition as defined in any one of Claims 1 to 4, in which the emulsifying agent or agents of the oil-in-water type are chosen from sorbitan oleate ethoxylated with 20 mol of ethylene oxide, ethoxylated castor oil comprising 40 mol of ethylene oxide, ethoxylated sorbitan laurate comprising 20 mol of ethylene oxide or ethoxylated lauryl alcohol comprising 7 mol of ethylene oxide.

6. Composition as defined in any one of Claims 1 to 5, in which the emulsifying agent or agents of the oil-in-water type are chosen from the compounds of formula (I) :
R₁-O-[CH(R₂)-CH₂-O]ₙ- (G)ₓ-H (I)
in which R₁ represents a saturated or unsaturated and linear or branched hydrocarbon radical comprising from 1 to 30 carbon atoms, R₂ represents a hydrogen atom or an alkyl radical comprising 1 or 2 carbon atoms, G represents the residue of a saccharide, x represents a decimal number between 1 and 5 and n is equal either to zero or to a number between 1 and 30.

7. Composition as defined in Claim 6, for which, in the formula (I), the number x is between 1 and 3, more particularly between 1.05 and 2.5, very particularly between 1.1 and 2.0 and preferably less than or equal to 1.5.

8. Composition as defined in either of Claims 6 and 7, for which, in the formula (I), G represents the glucose residue or the xylose residue and n is equal to 0.

9. Composition as defined in any one of Claims 6 to 8, for which, in the formula (I), R₁ represents a radical comprising from 8 to 18 carbon atoms and more particularly an octyl, decyl, undecyl, dodecyl, tetradecyl or hexadecyl radical, the said radicals being linear or branched.

10. Composition as defined in any one of Claims 1 to 9, for which the strong acid functional group of the monomer comprising it is the sulphonic acid functional group or the phosphonic acid functional group, partially or completely salified.

11. Composition as defined in Claim 10, for which the monomer comprising a strong acid functional group is 2-methyl-2-[(1-oxo-2-propenyl)amino]-1-propanesulphonic acid partially or completely salified in the sodium, potassium, ammonium or monoethanolamine salt form.

12. Composition as defined in any one of Claims 1 to 11, in which the polyelectrolyte is a copolymer comprising, in molar proportions, from 30% to 50% of 2-methyl-2-[(1-oxo-2-propenyl)amino]-1-propanesulphonic acid salified in the sodium salt or ammonium salt form and from 50% to 70% of acrylamide.

13. Composition as defined in any one of Claims 1 to 11, in which the polyelectrolyte is a homopolymer of 2-methyl-2-[(1-oxo-2-propenyl)amino]-1-propanesulphonic acid salified in the sodium salt or ammonium salt form.

14. Composition as defined in any one of Claims 1 to 13, **characterized in that** it comprises from 4% to 10% by weight of emulsifying agents.

15. Composition as defined in Claim 14, in which from 20% to 50% and more particularly from 25% to 40% of the total weight of the emulsifiers are of the water-in-oil type and from 80% to 50% and more particularly from 75% to 60% by weight are of the oil-in-water type.

16. Composition as defined in any one of Claims 1 to 15, **characterized in that** the oil phase represents from 15% to 40% and preferably from 20% to 25% of its total weight.

17. Composition as defined in any one of Claims 1 to 16, **characterized in that** it additionally comprises one or more additives chosen from complexing agents, transfer agents or chain-limiting agents.

18. Cosmetic, dermocosmetic, dermopharmaceutical or pharmaceutical composition, **characterized in that** it comprises from 0.1% to 10% by weight of the composition as defined in any one of Claims 1 to 16.

19. Cosmetic, dermocosmetic, dermopharmaceutical or pharmaceutical composition as defined in Claim 18, in the form of a milk, of a lotion, of a gel, of a cream, of a soap, of a foam bath, of a balm, of a shampoo or of a conditioner.

20. Use of a composition as defined in one of Claims 1 to 17 in preparing cosmetic, dermocosmetic, dermopharmaceutical or pharmaceutical compositions.

## Patentansprüche

1. Zusammensetzung, welche eine Ölphase, eine wässrige Phase, mindestens einen Emulgator des Typs Wasser-in-Öl (W/O) und mindestens einen Emulgator des Typs Öl-in-Wasser (O/W) umfasst und welche in Form eines selbstdispergierenden Inverslatex, bei dem die Phasenumkehr selbsttätig stattfinden kann, vorliegt und welche 20 bis 70 Gewichts%, und vorzugsweise 25 bis 50 Gew.%, eines verzweigten und/oder vernetzten Polyelektrolyten, in dem der molare Anteil einer doppelt oder mehrfach ethylenisch ungesättigten Verbindung bezogen auf die eingesetzten Monomere 0,005 % bis 1% und vorzugsweise 0,01 % bis 0,5 % sowie insbesondere 0,1 % bis 0,25 % beträgt, umfasst, **dadurch gekennzeichnet, dass** das Lösemittel, welches in der Ölphase enthalten ist, aus Squalan oder hydriertem Polyisobuten ausgewählt wird und dass es sich bei dem Polyelektrolyten
entweder um ein Homopolymer auf Basis eines Monomers, welches eine teilweise oder vollständig in die Salzform überführte starke funktionelle Säuregruppe aufweist, handelt oder aber um ein Copolymer auf Basis mindestens eines Monomers, welches eine teilweise oder vollständig in die Salzform überführte starke funktionelle Säuregruppe aufweist und welches mit Acrylamid sowie dem Vernetzungs- und/oder Verzweigungsmittel, das aus Diallyloxyessigsäure oder einem ihrer Salze wie etwa Natriumdiallyloxyacetat oder aus Ethylenglykoldimethacrylat, Ethylenglykoldiacrylat, Diallylharnstoff, Trimethylolpropantriacrylat, Methylenbis (acrylamid) oder Triallylamin gewählt wird, copolymerisiert ist.

2. Zusammensetzung gemäß Anspruch 1, **dadurch gekennzeichnet, dass** es sich bei dem Lösemittel, welches in der Ölphase enthalten ist, um hydriertes Polyisobuten handelt.

3. Zusammensetzung gemäß Anspruch 1, **dadurch gekennzeichnet, dass** es sich bei dem Lösemittel, welches in der Ölphase enthalten ist, um Squalan handelt.

4. Zusammensetzung gemäß einem der Ansprüche 1 bis 3, wobei der oder die Emulgatoren des Typs Wasser-in-Öl aus Sorbitanmonooleat, Sorbitanisostearat oder mit 5 Mol Ethylenoxid ethoxyliertem Sorbitanoleat ausgewählt werden.

5. Zusammensetzung gemäß einem der Ansprüche 1 bis 4, wobei der oder die Emulgatoren des Typs Öl-in Wasser aus mit 20 Mol Ethylenoxid ethoxyliertem Sorbitanoleat, mit 40 Mol Ethylenoxid ethoxyliertem Ricinusöl, mit 20 Mol Etylenoxid ethoxyliertem Sorbitanlaurat und mit 7 Mol Ethylenoxid ethoxyliertem Laurylalkohol ausgewählt werden.

6. Zusammensetzung gemäß einem der Ansprüche 1 bis 5, wobei der oder die Emulgatoren des Typs Öl-in-Wasser aus den Verbindungen nach Formel (I) ausgewählt werden:
R₁-O-[CH(R₂)-CH₂-O]ₙ-(G)ₓ-H (I)
wobei R₁ für ein geradkettiges oder verzweigtes, gesättigtes oder ungesättigtes Kohlenwasserstoffradikal mit 1 bis 30 Kohlenstoffatomen steht, R₂ für ein Wasserstoffatom oder ein Alkylradikal mit 1 oder 2 Kohlenstoffatomen steht, G für einen Saccharidrest steht, x für eine Dezimalzahl von 1 bis 5 steht und n entweder gleich null oder gleich einer Zahl von 1 bis 30 ist.

7. Zusammensetzung gemäß Anspruch 6, bei welcher die Zahl x in der Formel (I) zwischen 1 und 3, insbesondere zwischen 1,05 und 2,5, sowie ganz besonders zwischen 1,1 und 2,0 liegt und vorzugsweise kleiner oder gleich 1,5 ist.

8. Zusammensetzung gemäß einem der Ansprüche 6 oder 7, bei welcher G in der Formel (I) für den Glucoserest oder den Xyloserest steht und n gleich 0 ist.

9. Zusammensetzung gemäß einem der Ansprüche 6 bis 8, bei welcher R₁ in der Formel (I) für ein Radikal mit 8 bis 18 Kohlenstoffatomen und insbesondere für ein Octyl-, Decyl-, Undecyl-, Dodecyl-Tetradecyl- oder Hexadecylradikal steht, wobei die Radikale geradkettig oder verzweigt sein können.

10. Zusammensetzung gemäß einem der Ansprüche 1 bis 9, bei welcher es sich bei der starken funktionellen Säuregruppe, welche das Monomer aufweist, um eine teilweise oder vollständig in die Salzform überführte funktionelle Sulfonsäuregruppe oder funktionelle Phosphorsäuregruppe handelt.

11. Zusammensetzung gemäß Anspruch 10, bei welcher es sich bei dem Monomer mit starker funktioneller Säuregruppe um teilweise oder vollständig in die Salzform überführte 2-Methyl-2-[(1-oxo-2-propenyl)amino]-1-propansulfonsäure in Form von Natrium-, Kalium-, Ammonium- oder Monoethanolaminsalz handelt.

12. Zusammensetzung gemäß einem der Ansprüche 1 bis 11, wobei es sich bei dem Polyelektrolyten um ein Copolymer handelt, in welchem der Molanteil der 2-Methyl-2-[(1-oxo-2-propenyl)amino]-1-propansulfonsäure in Form von Natrium- oder Ammoniumsalz 30 % bis 50 % beträgt und derjenige des Acrylamids 50 % bis 70 %.

13. Zusammensetzung gemäß einem der Ansprüche 1 bis 11, wobei es sich bei dem Polyelektrolyten um ein Homopolymer der 2-Methyl-2-[(1-oxo-2-propenyl)amino]-1-propansulfonsäure, in Form von Natrium- oder Ammoniumsalz, handelt.

14. Zusammensetzung gemäß einem der Ansprüche 1 bis 13, **dadurch gekennzeichnet, dass** sie 4 bis 10 Gewichts% an Emulgatoren enthält.

15. Zusammensetzung gemäß Anspruch 14, wobei es sich bei 20 % bis 50 % und insbesondere bei 25 % bis 40 % des Gesamtgewichtes der Emulgatoren um solche des Typs Wasser-in-Öl und bei 80 % bis 50 % und insbesondere 75 bis 60 Gewichts % um solche des Typs Öl-in-Wasser handelt.

16. Zusammensetzung gemäß einem der Ansprüche 1 bis 15, **dadurch gekennzeichnet, dass** die Ölphase 15 % bis 40 % und vorzugsweise 20 % bis 25 % ihres Gesamtgewichtes ausmacht.

17. Zusammensetzung gemäß einem der Ansprüche 1 bis 16, **dadurch gekennzeichnet, dass** sie weiterhin einen oder mehrere Zusatzstoffe umfasst, welche aus den Komplexierungsmitteln, den Übertragungsmitteln oder den Mitteln zur Begrenzung der Kettenlänge gewählt werden.

18. Kosmetische, hautkosmetische, hautpharmazeutische oder pharmazeutische Zusammensetzung, **dadurch gekennzeichnet, dass** sie 0,1 bis 10 Gewichts% der Zusammensetzung gemäß einem der Ansprüche 1 bis 16 umfasst.

19. Kosmetische, hautkosmetische, hautpharmazeutische oder pharmazeutische Zusammensetzung gemäß Anspruch 18, welche in Form einer Milch, einer Lotion, eines Gels, einer Creme, einer Seife, eines Schaumbads, eines Balsams, eines Shampoos oder eines Nachbehandlungsmittels für die Haarwäsche vorliegt.

20. Verwendung einer Zusammensetzung gemäß einem der Ansprüche 1 bis 17, um kosmetische, hautkosmetische, hautpharmazeutische oder pharmazeutische Zusammensetzungen herzustellen.
